# EUROPÄISCHE PATENTANMELDUNG

(11) **EP 0 534 078 A1**
(43) Veröffentlichungstag der Anmeldung: **31.03.1993**
(21) Anmeldenummer: 92112407.9
(22) Anmeldetag: 20.07.1992
(51) Int. Cl.: A61C 8/00, A61K 6/04, A61K 6/027

(54) **Dentalimplantat**

(30) Priorität: 24.07.1991 DE 4124532
(71) Anmelder: IMPLA GmbH, D-61191 Rosbach (DE)
(72) Erfinder: Klaus, Gerold, W-7832 Kenzingen (DE)
(74) Vertreter: Lusuardi, Werther Giovanni, Dr.

(57) **Zusammenfassung**

Das Dentalimplantat dient zur Befestigung eines Zahnersatzes. Es weist einen, in einen Kieferknochen einschraubbaren, biokompatiblen Gewindeteil (1) und einen daran anschliessenden okklusalen Aufbauteil (2) zur Verankerung dentaler Suprakonstruktionen auf.
Der Aufbauteil (2) ist in seinem supragingivalen Abschnitt mit einer zahnsteinabweisenden Beschichtung (3) aus Gold versehen, welche im extraossären Bereich die Bildung von Plaque und damit von infektiösen Herden wirksam verhindert.

## Beschreibung

Die Erfindung bezieht sich auf ein Dentalimplantat zur Befestigung eines Zahnersatzes mit einem, in einen Kieferknochen einschraubbaren, biokompatiblen Gewindeteil und mit einem daran anschliessenden okklusalen Aufbauteil zur Verankerung dentaler Suprakonstruktionen.

Eine Vielzahl von gattungsgemässen Dentalimplantaten ist bereits bekannt, welche vorwiegend aus Titan oder ähnlichen biokompatiblen Stoffen bestehen. Um das Knochenwachstum um das Implantat herum zu fördern wird das Gewindeteil des Dentalimplantates meist mit einer aufgerauhten Reintitanoberfläche oder mit einer Titanplasmabeschichtung versehen, welche im oberen gingivalen und supragingivalen Bereich - in dem kein Knochenwachstum induziert werden muss - hochglanzpoliert ist. Der Nachteil dieser Reintitanoberfläche im supragingivalen Bereich, besteht gerade in ihrer Biokompatibilität, welche trotz Hochglanzpolitur zu einer hohen Anfälligkeit gegen Zahnsteinablagerungen führt. Die Bildung von Zahnsteinbelägen in diesem Übergangsbereich ist jedoch äusserst unerwünscht, weil dadurch bakterielle Infektionen auftreten können, welche zum Verlust des Implantats führen.

Hier will die Erfindung Abhilfe schaffen. Der Erfindung liegt die Aufgabe zugrunde, ein Dentalimplantat zu schaffen, welches aus langjährig erprobten, biokompatiblen Materialien gefertigt werden kann, welches jedoch die Bildung von Plaque und damit von infektiösen Herden wirksam verhindert.

Die Erfindung löst die gestellte Aufgabe mit einem Dentalimplantat, bei dem der Aufbauteil mindestens in seinem supragingivalen Abschnitt mit einer zahnsteinabweisenden Beschichtung versehen ist, welche zu einer der folgenden Materialgruppen gehört:
A) Gold, Platin, Iridium, Osmium oder einer Legierung dieser Metalle;
B) keramische Stoffe; oder
C) diamantartiger Kohlenstoff (DLC "diamond-like carbon").

Die Dicke dieser zahnsteinabweisenden Beschichtungen kann innerhalb gewisser Grenzen variieren, vorzugsweise liegt sie jedoch im Ångström bis µm Bereich.

Die für die zahnsteinabweisenden Beschichtungen verwendbaren keramischen Stoffe sind bevorzugt dentalkeramische Stoffe, wie sie im Medizin-Wörterbuch von Zetkin/Schaldach, 7.Aufl., 1985, Verlag Georg Thieme definiert sind. In diesem Falle liegt die Schichtdicke vorzugsweise im Millimeterbereich.

Die zahnsteinabweisenden Beschichtungen können entweder auf den Aufbauteil des Dentalimplantates aufgeklebt werden oder galvanisch darauf aufgebracht werden.
Beschichtung, die zur Materialgruppe A) gehören, können vorzugsweise galvanisch auf den Aufbauteil aufgebracht werden.

Das Gewindeteil besteht vorteilhafterweise, mindestens an seiner unbeschichteten Oberfläche, aus Reintitan, vorzugsweise in aufgerauhter Form.
Es ist auch möglich den gesamten Aufbauteil mit der erfindungsgemässen Beschichtung zu versehen.

Die durch die Erfindung erreichten Vorteile sind im wesentlichen darin zu sehen, dass dank des erfindungsgemässen Dentalimplantates die Hygienefreundlichkeit erhöht und insbesondere die Bildung von Zahnsteinbelägen im supragingivalen Bereich verhindert wird, was zu einer Reduktion der Infektionsrate führt.

Ein Ausführungsbeispiel der Erfindung ist in der Zeichnung dargestellt und wird im folgenden näher beschrieben.

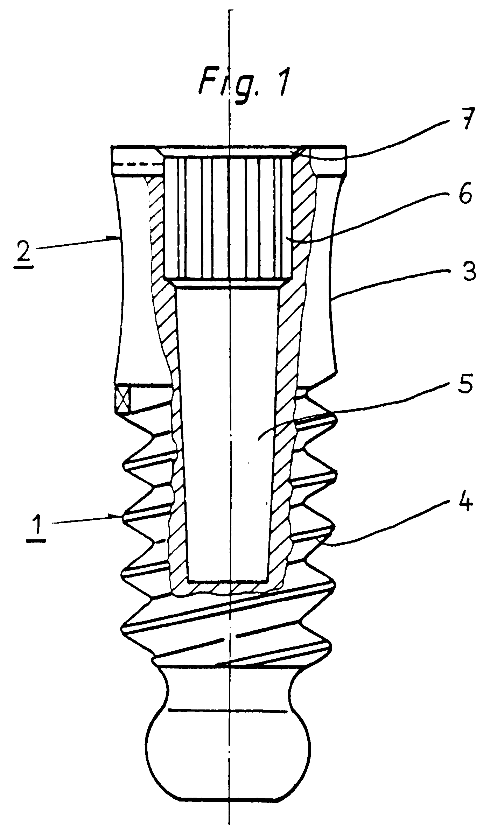

Fig. 1 stellt eine partielle Längsschnittdarstellung durch das erfindungsgemässe Dentalimplantat dar.

Das in Fig. 1 gezeigte Dentalimplantat besteht im wesentlichen aus einem, in einen Kieferknochen einschraubbaren Gewindeteil 1 und einem daran anschliessenden okklusalen Aufbauteil 2. Das Dentalimplantat besteht aus Reintitan, welches im Bereich des Gewindeteils 1 zur Erhöhung der Biokompatibilität aufgerauht ist. Der Aufbauteil 2 ist in seinem extraossären Bereich mit einer galvanisch aufgebrachten Goldschicht versehen. Im übrigen ist der Gewindeteil 1 mit einem konventionellen Gewinde 4 versehen, welches transgingival inseriert werden kann.

Der Aufbauteil 2 ist mit einer axialen Vertiefung 5 versehen, welche eine Mehrkantaufnahme 6 mit Anfasung 7 aufweist, in welche einerseits ein geeignetes Instrument eingeführt werden kann, um das Dentalimplantat in den Knochen eindrehen zu können, und in dem anderseits - zeichnerisch nicht dargestellte - dentale Suprakonstruktionen, beispielsweise Zahnprothesen, verankert werden können. Dazu geeignete Verankerungsvorrichtungen sind beispielsweise im DE-U1 90.12.548.7 beschrieben.

## Patentansprüche

1. Dentalimplantat zur Befestigung eines Zahnersatzes mit einem, in einen Kieferknochen einschraubbaren, biokompatiblen Gewindeteil (1) und mit einem daran anschliessenden okklusalen Aufbauteil (2) zur Verankerung dentaler Suprakonstruktionen, dadurch gekennzeichnet, dass der Aufbauteil (2) mindestens in seinem supragingivalen Abschnitt mit einer zahnsteinabweisenden Beschichtung (3) versehen ist, welche zu einer der folgenden Materialgruppen gehört:
A) Gold, Platin, Iridium, Osmium oder einer Legierung dieser Metalle;
B) keramische Stoffe; oder
C) diamantartiger Kohlenstoff (DLC "diamond-like carbon").

2. Dentalimplantat nach Anspruch 1, dadurch gekennzeichnet, dass der keramische Stoff ein dentalkeramischer Stoff ist.

3. Dentalimplantat nach Anspruch 1 oder 2, dadurch gekennzeichnet, dass die Beschichtung (3) auf dem Aufbauteil (1) aufgeklebt ist.

4. Dentalimplantat nach Anspruch 1, dadurch gekennzeichnet, dass die Beschichtung (3) zur Materialgruppe A) gehört und galvanisch auf den Aufbauteil (1) aufgebracht ist.

5. Dentalimplantat nach einem der Ansprüche 1 - 4, dadurch gekennzeichnet, dass das Gewindeteil (1) mindestens an seiner unbeschichteten Oberfläche aus Reintitan, vorzugsweise in aufgerauhter Form, besteht.

6. Dentalimplantat nach einem der Ansprüche 1 - 5, dadurch gekennzeichnet, dass das gesamte Aufbauteil (2) mit der Beschichtung (3) versehen ist.
